(19) Europäisches Patentamt
European Patent Office
Office européen des brevets

(11) Publication number : **0 404 356 B1**

(12) # EUROPEAN PATENT SPECIFICATION

(45) Date of publication of patent specification : **22.09.93 Bulletin 93/38**

(51) Int. Cl.5 : **C07D 495/04,** A61K 31/505, // (C07D495/04, 239:00, 333:00)

(21) Application number : **90305636.4**

(22) Date of filing : **23.05.90**

(54) **Substituted thienopyrimidine derivatives, their preparation, pharmaceutical compositions and medical use.**

(30) Priority : **30.05.89 GB 8912335**

(43) Date of publication of application : **27.12.90 Bulletin 90/52**

(45) Publication of the grant of the patent : **22.09.93 Bulletin 93/38**

(84) Designated Contracting States : **CH DE FR GB IT LI NL**

(56) References cited :
**EP-A- 0 082 023**
**EP-A- 0 150 469**
**EP-A- 0 276 057**
**DE-A- 2 200 764**
**GB-A- 1 309 182**
**US-A- 4 146 716**

(73) Proprietor : **SMITHKLINE BEECHAM INTERCREDIT B.V.**
**Jaagpad 1 P.O. Box 3120**
**NL-2280 GC Rijswijk (NL)**

(72) Inventor : **Brown, Thomas Henry**
**SmithKline & French Res. Ltd., The Frythe, Welwyn, Hertfordshire AL6 9AR (GB)**
Inventor : **Ife, Robert John**
**SmithKline & French Res. Ltd., The Frythe, Welwyn, Hertfordshire AL6 9AR (GB)**
Inventor : **Leach, Colin Andrew**
**SmithKline & French Res. Ltd., The Frythe, Welwyn, Hertfordshire AL6 9AR (GB)**

(74) Representative : **Giddings, Peter John, Dr. et al**
**SmithKline Beecham, Corporate Patents, Mundells**
**Welwyn Garden City, Hertfordshire AL7 1EY (GB)**

EP 0 404 356 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted thienopyrimidine derivatives, processes for their preparation, intermediates useful in their preparation, pharmaceutical compositions containing them and their use in therapy.

DE-A-2 200 764 (Karl Thomae) discloses a series of thieno[2,3-d]pyrimidine derivatives having an amino group at the 2-position, and a cyclic group such as morpholino at the 4-position. These compounds are said to inhibit platelet aggregation.

GB 1 309 182 (Karl Thomae) discloses thieno[3,2-d]pyrimidines in which the 2- and 4-positions are substituted by piperazine and morpholine groups. These compound also have utility as platelet aggregation inhibitors.

Related thieno[2,3-d]pyrimidines are disclosed in EP-A-82 023 (Sankyo) in which the 2-position may be substituted by heterocyclic moieties such as piperazine and thiomorpholine groups, and the 4-position may be substituted with an amino group. These compounds are alleged to have various utilities including the treatment of thrombosis.

A further series of thienopyrimidines is disclosed in US 4 146 716 (ICI) having utility as fungicides and pesticides.

EP-A-276 057 (Merck) discloses thieno- and thiopyranopyrimidines substituted with a piperazine group in the 4-position. These compounds are β-adrenergic receptor blockers.

Accordingly the present invention provides, in a first aspect compounds of structure (I)

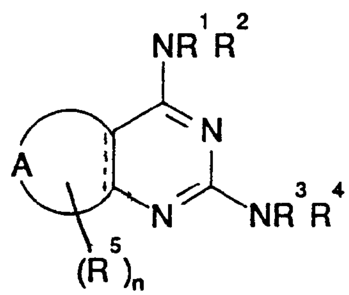

(I)

in which

$R^1$ and $R^2$ are the same, or different and are each hydrogen, $C_{1-4}$alkyl, $-(CH_2)_nAr$ in which n is 0 to 4 and Ar is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl;

$R^3$ and $R^4$ are the same or different and are each hydrogen, $C_{1-4}$alkyl, $(CH_2)_nAr^1$ in which n is 0 to 4 and $Ar^1$ is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl;

$R^5$ is hydrogen, $C_{1-4}$alkyl, $C_{1-4}$alkoxy or $COC_{1-4}$alkyl;

n is 1 or 2;

A is -SCH=CH-, -CH=CHS- or =CHSCH=, and the dotted line indicates the presence of a double bond when A is -SCH=CH- or -CH=CHS-;

and pharmaceutically acceptable salts thereof; with the exception of 2-isobutylamino-4-isopropylaminothieno[2,3-d]pyrimidine.

Suitably $R^1$ and $R^2$ are the same or different and are each hydrogen or $(CH_2)_nAr$ in which n is 0 to 4 and Ar is an optionally substituted phenyl group. More suitably, one of $R^1$ and $R^2$ is hydrogen or $C_{1-4}$alkyl and the other is hydrogen, $C_{1-4}$alkyl or $(CH_2)_nAr$. Most suitably one of $R^1$ and $R^2$ is hydrogen or $C_{1-4}$alkyl and the other is $(CH_2)_nAr$. Preferably one of $R^1$ and $R^2$ is $C_{1-4}$alkyl and the other is $(CH_2)_nAr$; most preferably one of $R^1$ and $R^2$ is $C_{1-4}$alkyl, in particular methyl and the other is $(CH_2)_nAr$ in which n is 0.

Suitably, Ar is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More suitably, Ar is unsubstituted or substituted by two substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More preferably Ar is unsubstituted or substituted by two substituents selected from $C_{1-4}$alkyl and $C_{1-4}$alkoxy. Most preferably, Ar is unsubstituted or substituted by a single substituent selected from the above-noted groups, in particular $C_{1-4}$alkyl or $C_{1-4}$alkoxy.

Suitably, $R^3$ and $R^4$ are the same or different and are each hydrogen, $C_{1-4}$alkyl, $(CH_2)_nAr^1$ in which n is 0 to 4 and $Ar^1$ is an optionally substituted phenyl group. More suitably one of $R^3$ and $R^4$ is hydrogen or $C_{1-4}$alkyl and the other is hydrogen, $C_{1-4}$alkyl or $(CH_2)_nAr^1$. Most suitably, one of $R^3$ and $R^4$ is hydrogen or $C_{1-4}$alkyl and the other is hydrogen, $C_{1-4}$alkyl or $(CH_2)_nAr^1$. Preferably one of $R^3$ and $R^4$ is hydrogen or $C_{1-4}$alkyl and the other is $(CH_2)_nAr^1$; more preferably one of $R^3$ and $R^4$ is hydrogen and the other is $(CH_2)_nAr^1$; most preferably, one of $R^3$ and $R^4$ is hydrogen and the other is $(CH_2)_nAr^1$ in which n is O.

Suitably, the group $Ar^1$ is unsubstituted or optionally substituted by 1 to 3 substituents as hereinabove described for the group Ar in $R^1$ or $R^2$. Preferably the group $Ar^1$ is substituted by one or two groups, for example a $C_{1-4}$alkyl group, in particular a methyl group or a halogen atom, in particular a fluorine atom; or a $C_{1-4}$alkyl group and a halogen atom or $C_{1-4}$alkoxy group in particular a methyl group and fluorine atom or methoxy group and fluorine atom. More preferably the group $Ar^1$ is substituted by a single group in the 2-position of the ring in particular, a methyl group.

Suitably $R^5$ is hydrogen, $C_{1-4}$alkyl, $C_{1-4}$alkoxy or $COC_{1-4}$alkyl; preferably $R^5$ is hydrogen.

Suitably n is 1 or 2; preferably n is 1.

Suitably, A is =CHSCH=, -CH=CHS- or -SCH=CH-; preferably A is -SCH=CH-.

$C_{1-4}$alkyl groups (either alone or as part of another group) can be straight or branched.

It will be appreciated that compounds of structure (I) in which one or more of $R^1$ to $R^5$ is a $C_{3-4}$alkyl group (either alone or as part of another group) may contain an assymetric centre due to the presence of the $C_{3-4}$alkyl group. Such compounds will exist as two (or more) optical isomers (enantiomers). Both the pure enantiomers, racemic mixtures (50% of each enantiomer) and unequal mixtures of the two are included within the scope of the present invention. Further, all diastereomeric forms possible (pure enantiomers and mixtures thereof) are within the scope of the invention.

The compounds of the present invention can be prepared by processes analogous to those known in the art. The present invention therefore provides in a further aspect a process for the preparation of a compound of structure (I) or a pharmaceutically acceptable salt thereof which comprises

(a) reaction of a compound of structure (II)

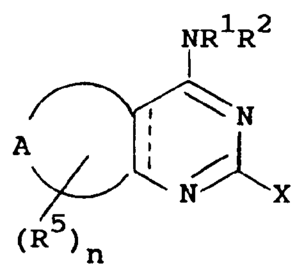

(II)

in which $R^1$, $R^2$, $R^5$, A and n are as described for structure (I) except that where necessary they are in protected form, and X is a group displaceable by an amine, with an amine of structure $R^3R^4NH$ in which $R^3$ and $R^4$ are as described for structure (I);

(b) reaction of a compound of structure (III)

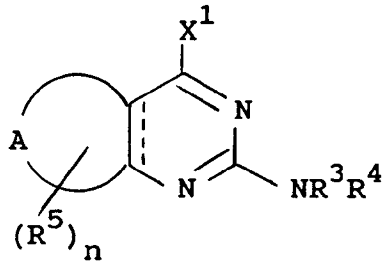

(III)

in which $R^3$, $R^4$, $R^5$, A and n are as described for structure (I) and $X^1$ is a group displaceable by an amine,

with an amine of structure $R^1R^2NH$ in which $R^1$ and $R^2$ are as described for structure (I); or

(c) for compounds of structure (I) in which $NR^1R^2$ and $NR^3R^4$ are the same, reaction of a compound of structure (IV)

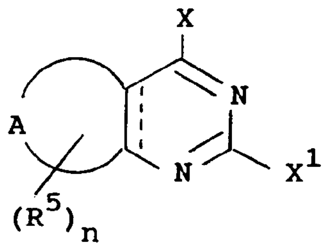

(IV)

in which A, $R^5$ and n are as described for structure (I) and X and $X^1$ are groups displaceable by an amine, with an amine of structure $R^1R^2NH$ or $R^3R^4NH$

and optionally thereafter,

° removing any protecting groups;

° forming a pharmaceutically acceptable salt.

Suitable groups displaceable by an amine, X and $X^1$, will be apparent to those skilled in the art and include, for example, halogen, in particular chlorine, $SC_{1-4}$alkyl, such as methylthio, hydroxy and phenoxy.

Reaction of a compound of structure (II) with an amine $R^3R^4NH$ is suitably carried out in an inert solvent at elevated temperature. Preferably the reaction is carried out in the absence of a solvent at elevated temperature.

Reaction of a compound of structure (III) with an amine $R^1R^2NH$ is suitably carried out in the presence or absence of an inert solvent, preferably in the absence of a solvent.

Reaction of a compound of structure (IV) with a suitable amine is suitably carried out under similar conditions to those described for the reaction of a compound of structure (III) and an amine of structure $R^1R^2NH$.

In particular, leaving groups X and $X^1$ are halogen, preferably chlorine, and can be displaced by appropriate amines $R^1R^2NH$ and $R^3R^4NH$ under the general conditions described above and in the specific examples. Other conditions and reagents depending on the nature of the leaving groups will be apparent to those skilled in the art; for example compounds of structure (I) in which $R^1$ and $R^2$ are both hydrogen, can be prepared from the corresponding compounds of structure (III) in which X is hydroxy by reaction with phenylphosphordiamidate using the method described in J. Het. Chem (1972), 9, 1235.

Pharmaceutically acceptable acid addition salts of the compounds of structure (I) can be prepared by standard procedures by, for example, reaction with suitable organic and inorganic acids the nature of which will be apparent to persons skilled in the art. For example, pharmaceutically acceptable salts can be formed by reaction with hydrochloric, sulphuric, or phosphoric acids; aliphatic, aromatic or heterocyclic sulphonic acids or carboxylic acids such as, for example, citric, maleic or fumaric acids.

The intermediate compounds of structure (II) and (III) can be prepared by procedures analogous to those known in the art. The amines of structure $R^1R^2NH$ and $R^3R^4NH$ are available commercially or can be prepared by standard techniques well known to those skilled in the art of organic chemistry.

For example compounds of structure (II) in which A is -CH=CHS- or -SCH=CH- and X is chlorine can be prepared by the route outlined in Scheme I.

Scheme I

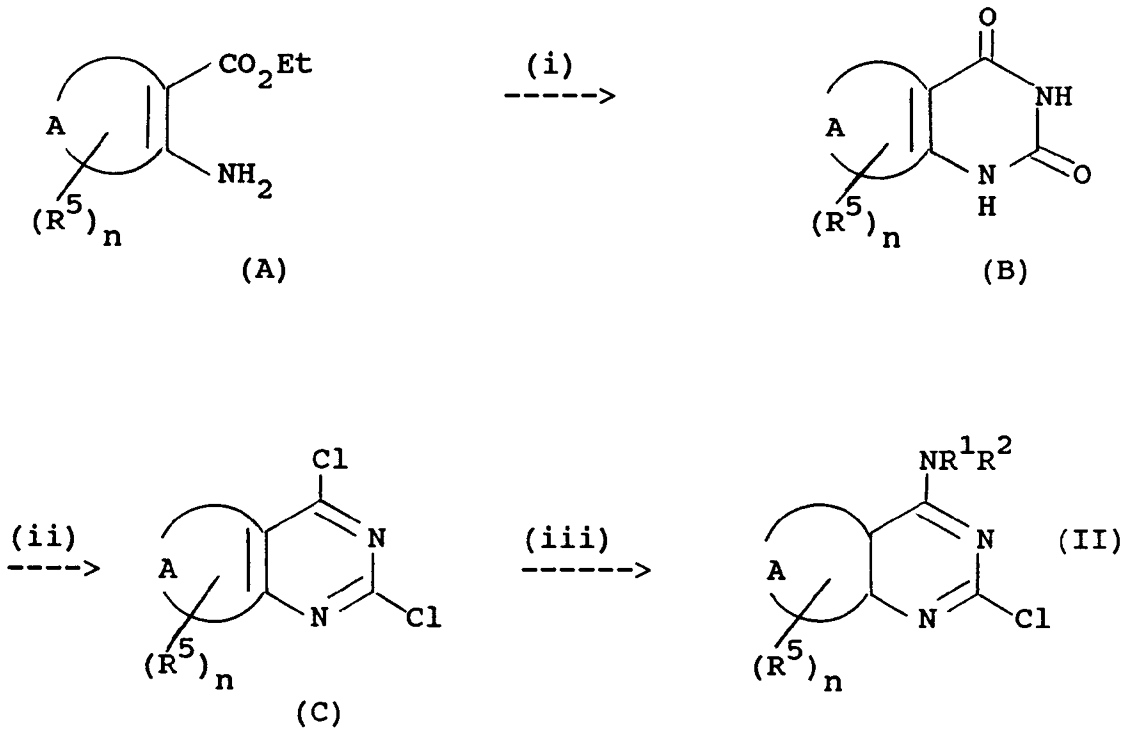

(A) (B) (C) (II)

(i) Urea

(ii) $POCl_3$, $PhNMe_2$, $\Delta$

(iii) $R^1R^2NH$, EtOH.

Compounds of structure (III) in which A is -SCH=CH- and X is chlorine can be prepared by the procedures outlined in Scheme II.

Scheme II

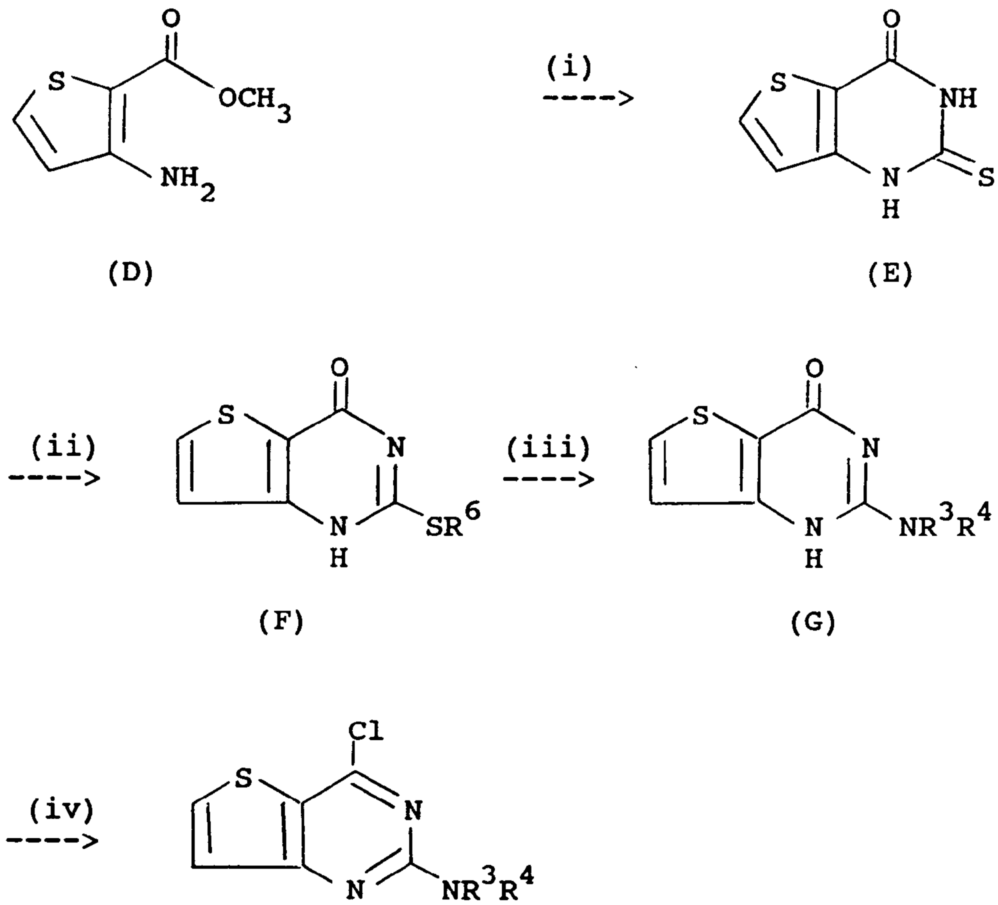


(D) (E)

(F) (G)

(III)

(i) KSCN, HCl

(ii) $R^6X^2$, NaOH ($R^6$ = $C_{1-4}$alkyl, $X^2$ = halogen)

(iii) $R^3R^4NH$,

(iv) $POCl_3$, Δ

Compounds of structure (III) in which A is =CH-SCH= can be prepared via the reactions outlined in Scheme III below

Scheme III

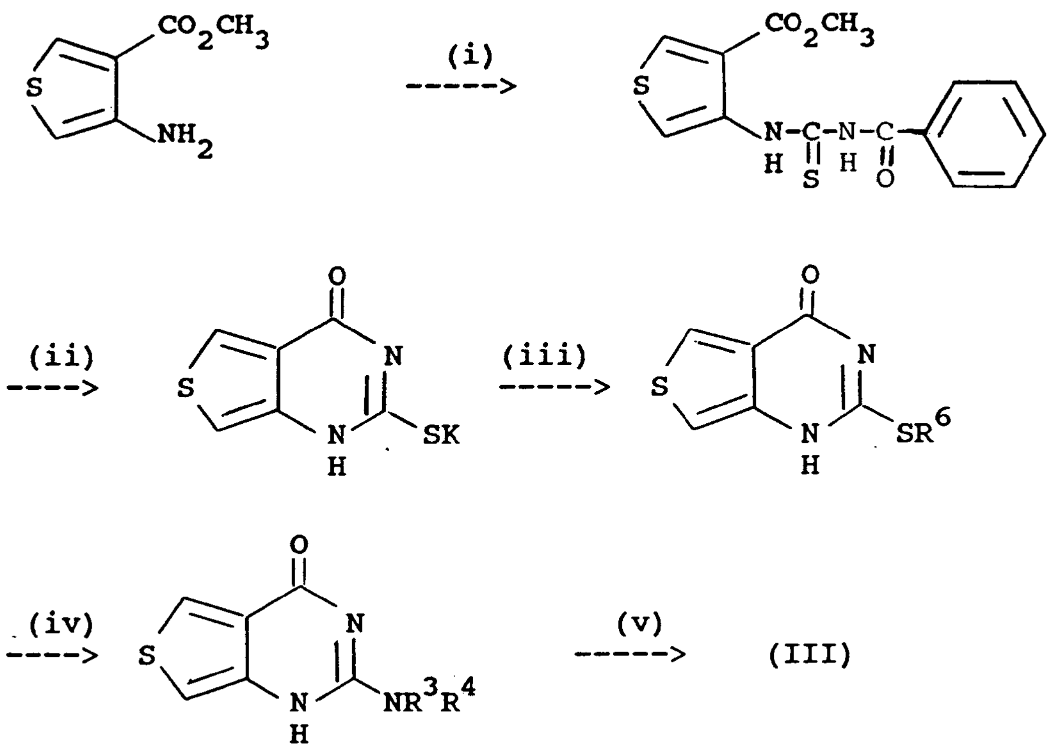


(i) benzoyl isothiocyanate, toluene

(ii) KOH, MeOH

(iii) $R^6X^2$ ($R^6$ = $C_{1-4}$alkyl, $X^2$ = halogen)

(iv) $HNR^3R^4$,

(v) $POCl_3$, Δ

The starting materials used to prepare compounds of structures (II) and (III) are available commercially or can be prepared by standard techniques.

In particular compounds (C) in Scheme I in which A is CH=CHS and $R^5$ is hydrogen are described in GB 1,570,494 and Bull. Soc. Chim. France 592, 1975; compounds (C) in which A is SCH=CH and $R^5$ is hydrogen are described in DE 2,058,086; and compounds (F) from Scheme II in which $R^6$ is ethyl are disclosed in GB 1,309,182.

It is to be noted, and apparent to those skilled in the art that in the foregoing reactions, where necessary groups on aromatic rings Ar and $Ar^1$ (e.g. hydroxy or amino groups) will be in "protected" form. For example, amino groups can be "protected" in the form of nitro groups and converted into amino groups as appropriate, and hydroxy groups can be protected using standard groups for example as described in "Greene, T.W., Protective Groups in Organic Chemistry" which also provides examples of further appropriate protective groups for other moities.

The compounds of the invention and their pharmaceutically acceptable salts exert an anti-secretory effect by inhibition of the gastrointestinal $H^+K^+$ATPase enzyme (Fellenius E., Berglindh T., Sachs G., Olke L., Elander

B., Sjostrand S.E., and Wallmark B., 1981, Nature, **290**, 159-61).

In a further aspect therefore the present invention provides compounds of structure (I) and pharmaceutically acceptable salts thereof for use in therapy.

The compounds of structure (I) and their pharmaceutically acceptable salts inhibit exogenously and endogenously stimulated gastric acid secretion and are useful in the treatment of gastrointestinal diseases in mammals, in particular humans. Such diseases include, for example, gastric and duodenal ulcers, and Zollinger-Ellison Syndrome. Further, the compounds of structure (I) can be used In the treatment of other disorders where an anti-secretory effect is desirable for example in patients with gastritis, NSAID induced gastritis, gastric ulcers, acute upper intestinal bleeding, in patients with a history of chronic and excessive alcohol consumption, and in patients with gastro oesophageal reflux disease (GERD).

In addition to the foregoing use the compounds of structure (I) can be expected to be of use in medicine as inhibitors of bone resorption. In normal subjects there is a balance between bone resorption and bone formation, however in subjects with bone affected diseases such as osteoporosis, Paget's disease and hyperparathyroidism and related disorders this balance is disturbed. As a consequence the subject suffers a loss of bone tissue, decreased bone mass and bone fragility which can result in fracturing of bones. Bone resorption (or bone loss) is associated with the activity of osteoclast cells, and it is thought that agents which inhibit the activity of such cells (and so inhibit bone resorption) will have a beneficial effect on the reduction of bone loss and be of benefit in the treatment of the above-noted disease states. The present compounds can be expected to be inhibitors of osteoclast activity and bone resorption and to be of use in medicine in the treatment of diseases in which bone loss is a factor, in particular osteoporosis, Paget's disease and hyperparathyroidism.

The invention therefore provides for the use of a compound of formula (IA):

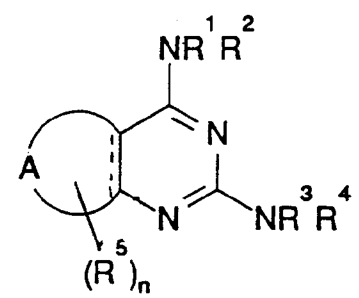

(IA)

in which

$R^1$ and $R^2$ are the same, or different and are each hydrogen, $C_{1-4}$alkyl, $-(CH_2)_nAr$ in which n is 0 to 4 and Ar is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^1$ and $R^2$ together with the nitrogen atom to which they are attached form a saturated or unsaturated ring optionally containing one or more further heteroatoms; and;

$R^3$ and $R^4$ are the same or different and are each hydrogen, $C_{1-4}$alkyl, $(CH_2)_nAr^1$ in which n is O to 4 and $Ar^1$ is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^3$ and $R^4$ together with the nitrogen atom to which they are attached form a saturated or unsaturated ring optionally containing one or more further heteroatoms.

$R^5$ is hydrogen, $C_{1-4}$alkyl, $C_{1-4}$alkoxy or $COC_{1-4}$alkyl;

n is 1 or 2;

A is -SCH=CH-, -CH=CHS- or =CHSCH=, and the dotted line indicates the presence of a double bond when A is -SCH=CH- or -CH=CHS-;

and pharmaceutically acceptable salts thereof in the manufacture of a medicament for the inhibition of gastric acid secretion or bone resorption.

In therapeutic use, the compounds of the present invention are usually administered in a standard pharmaceutical composition. The present invention therefore provides in a further aspect pharmaceutical compositions comprising a compound of structure (I) or a pharmaceutically acceptable salt thereof and a pharmaceutically acceptable carrier.

The compounds of structure (I) and their pharmaceutically acceptable salts which are active when given

orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base.

The present invention also provides a method of inhibiting gastric acid secretion which comprises administering to a mammal in need thereof an effective amount of a compound of structure (I) or a pharmaceutically acceptable salt thereof; and a method of treatment of diseases of the stomach or intestine based on increased acid secretion which comprises administering to a mammal in need thereof an effective amount of a compound of structure (I) or a pharmaceutically acceptable salt thereof.

The pharmaceutically acceptable compounds of the invention will normally be administered to a subject for the treatment of gastrointestinal diseases and other conditions caused or exacerbated by gastric acidity. The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co-administered with further active ingredients, such as antacids (for example magnesium carbonate or hydroxide and aluminium hydroxide), non-steroidal anti-flammatory drugs (for example indomethacin, aspirin or naproxen), steroids, or nitrite scavengers (for example ascorbic acid or aminosulphonic acid), or other drugs used for treating gastric ulcers (for example pirenzipine, prostanoids for example 16,16 dimethyl $PGE_2$, or histamine $H_2$-antagonists (for example, cimetidine).

The following examples illustrate the invention. Temperatures are recorded in degrees centigrade.

## THIENO[3,2-d]-PYRIMIDINES

### (a) 2-(2-Methylphenylamino)-4-hydroxythieno[3,2-d]pyrimidine

2-Ethylmercapto-4-hydroxythieno[3,2-d]pyrimidine (10 g, 0.0471 mol) and o-toluidine (30 g, 0.0279 mol) were heated in an oil bath at 210° for 20 hours. The reaction mixture was poured into diethyl ether and the solid obtained was collected by filtration and dried. The mother liquor was extracted with 2N sodium hydroxide (3 x 100 ml). The aqueous extracts were combined, acidified with glacial acetic acid and extracted with chloroform (3 x 100 ml). The chloroform extracts were combined, dried over magnesium sulphate, filtered and evaporated under reduced pressure to give a solid. Recrystallization from methanol gave a pure sample, (5.32 g), m.p. 243-245°.

$C_{13}H_{11}N_3OS$

Found C 60.82, H 4.36, N 16.64, S 12.02%;
Requires C 60.68, H 4.31, N 16.33, S 12.46%.

The following compounds were made in a similar manner.

2-(4-Methoxy-2-methylphenylamino)-4-hydroxythieno[3,2-d]pyrimidine

m.p.246-248° (methanol).

$C_{14}H_{13}N_3O_2S$

Found C 58.46, H 4.54, N 14.52, S 11.46%;
Requires C 58.52, H 4.56, N 14.62, S 11.16%.

2-(4-Fluoro-2-methylphenylamino)-4-hydroxythieno[3,2-d]pyrimidine

m.p. 250-252° (methanol).

(b) 2-(2-methylphenylamino)-4-chlorothieno[3,2-d]pyrimidine

2-(2-Methylphenylamino)-4-hydroxythieno[3,2-d]pyrimidine (2 g, 0.0078 mol) and phosphorus oxychloride (20 ml) were heated under reflux for 1 hour. The solution was poured onto ice, basified with concentrated $NH_3$ solution, and extracted with dichloromethane (3 x 100 ml). The dichloromethane extracts were combined, dried over magnesium sulphate and evaporated to give a yellow solid, 2.08 g. Recrystallization from methanol gave an analytically pure sample, m.p. 87-89°.

$C_{13}H_{10}ClN_3S$

Found C 56.46, H 3.62, N 15.20, Cl 12.77, S 12.02%;
Requires C 56.62, H 3.66, N 15.24, Cl 12.86, S 11.63%.

The following compounds were prepared in a similar manner.

2-(4-Methoxy-2-methylphenylamino)-4-chlorothieno[3,2-d]pyrimidine

m.p. 151-153° (methanol)

$C_{14}H_{12}ClN_3OS$

Found C 54.82, H 4.13, N 13.68, Cl 11.23, S 10.65%;
Requires C 54.99, H 3.96, N 13.74, Cl 11.59, S 10.49%.

2-(4-Fluoro-2-methylphenylamino)-4-chlorothieno[3,2-d]pyrimidine

m.p. 162-164° (methanol)

$C_{13}H_9ClFN_3S$

Found C 52.92, H 3.33, N 14.09, Cl 12.08, S 10.97%;
Requires C 53.15, H 3.09, N 14.31, Cl 12.07, S 10.92%.

Example 1

2-(2-Methylphenylamino)-4-(N-methylphenylamino)thieno[3,2-d]pyrimidine.

2-(2-Methylphenylamino)-4-chlorothieno[3,2-d]pyrimidine (1.5 g, 0.0054 mol) and N-methylaniline (1.16 g, 0.0054 mol) were heated in an oil bath at 140° for 2 hours. The reaction mixture was diluted with dichloromethane (100 ml) and washed with 2N hydrochloric acid (3 x 100 ml), then sodium carbonate solution. The dichloromethane extracts were dried over magnesium sulphate, filtered and evaporated to give a solid. Recrystallization from methanol gave the title compound, (0.75 g), m.p. 198-200°.

$C_{20}H_{18}N_4S$

Found C 69.37, H 5.38, N 16.17, S 9.15%;

Requires C 69.34, H 5.24, N 16.17, S 9.26%.

Example 2

2-(4-Methoxy-2-methylphenylamino)-4-(N-methylphenylamino)thieno[3,2-d]pyrimidine hydrochloride.

2-(4-Methoxy-2-methylphenylamino)-4-chlorothieno[3,2-d]pyrimidine (4 g, 0.013 mol) and N-methylaniline (2.8 g, 0.026 mol) were heated at 140° for 1 hour. The addition of diethyl ether gave a solid which was collected by filtration and dried, (5.5 g). Recrystallization from ethanol/diethyl ether gave the title compound, (4.59 g), m.p. 225-227°.

$C_{21}H_{20}N_4OS$ HCl

Found C 61.05, H 5.25, N 13.66, $Cl^-$ 8.53, S 7.72%;

Requires C 61.08, H 5.13, N 13.57, $Cl^-$ 8.59, S 7.77%.

Example 3

2-(4-Fluoro-2-methylphenylamino)-4-(N-methylphenylamino)thieno[3,2-d]pyrimidine hydrochloride

2-(4 Fluoro-2-methylphenylamino)-4-chlorothieno[3,2-d]pyrimidine (1.5 g, 0.0051 mol) and N-methylaniline were heated in an oil bath at 150° for 1 hour. Addition of diethyl ether gave a solid which was collected by filtration and dried, (1.85 g). Recrystallization from ethanol gave the title compound, (0.75 g), m.p. 242-244°.

$C_{20}H_{17}FN_4S$ HCl

Found C 60.18, H 4.35, N 14.14, $Cl^-$ 8.64, S 8.19%;

Requires C 59.92, H 4.53, N 13.98, $Cl^-$ 8.84, S 8.00%.

Example 4

2,4-Bis(N-methylphenylamino)thieno[3,2-d]pyrimidine

2,4-Dichlorothieno[3,2-d]pyrimidine (1 g, 0.0048 mol) and N-methylaniline (5 ml) were heated at 130° for 1 hour. The reaction mixture was diluted with dichloromethane (200 ml) and extracted with 2N hydrochloric acid (3 x 150ml). The organic phase was then washed with sodium carbonate, dried over magnesium sulphate, filtered and evaporated under reduced pressure to give a brown solid. Recrystallization from methanol gave the title compound, (1.08 g), m.p. 120-122°.

$C_{20}H_{18}N_4S$

Found C 69.51, H 5.02, N 16.21, S 9.32%;
Requires C 69.34, H 5.24, N 16.17, S 9.26%.

THIENO[2,3-d]-PYRIMIDINES

(a) 2-Chloro-4-(N-methylphenylamino)thieno[2,3-d]pyrimidine

2,4-Dichlorothieno[2,3-d]pyrimidine (4.5 g, 0.022 mol) and N-methylaniline (4.7 g, 0.044 mol) in ethanol (50 ml) were stirred at room temperature for 72 hours. The solid obtained was collected by filtration, washed with ethanol and dried, 5.15 g. Recrystallization from ethyl acetate gave the title compound, m.p. 167-168°

$C_{13}H_{10}ClN_3S$

Found C 56.72, H 3.75, N 15.25, Cl 12.99, S 11.84%;
Requires C 56.62, H 3.66, N 15.24, Cl 12.86, S 11.63%.

(b) 2-Chloro-4-(phenylamino)thieno[2,3-d]pyrimidine

2,4-Dichlorothieno[2,3-d]pyrimidine (3.2 g, 0.0156 mol) and aniline (2.9 g, 0.0311 mol) in ethanol were stirred at room temperature for 20 hours. The solvent was evaporated under reduced pressure to give an oil. The oil was treated with diethyl ether to give a solid, aniline hydrochloride. The filtrate was evaporated under reduced pressure to give the product as a solid, (3.74 g). Recrystallization (0.7 g) from methanol gave a pure sample of the title compound, (0.53 g), m.p. 149-151°.

Example 5

2-(2-Methylphenylamino)-4-(N-methylphenylamino)thieno[2,3-d]pyrimidine hydrochloride.

2-Chloro-4-(N-methylphenylamino)thieno[2,3-d]pyrimidine (1.5 g, 0.0054 mol) and o-toluidine (1.3 g, 0.012 mol) were heated at 160° in an oil bath for 2 hours. The addition of methanol gave a solid which was collected by filtration and dried. Recrystallization from ethanolic HCl gave the title compound as a partial hydrochloride salt, (1.13 g), m.p. 210-212°.

$C_{20}H_{18}N_4S$ 0.65HCl

Found C 64.92, H 5.17, N 15.04, S 8.47, $Cl^-$ 6.51%;
Requires C 64.89, H 5.08, N 15.14, S 8.66, $Cl^-$ 6.23%.

Example 6

2-(4-Fluoro-2-methylphenylamino)-4-(N-methylphenylamino)thieno-[2,3-d]pyrimidine hydrochloride

2-Chloro-4-(N-methylphenylamino)thieno[2,3-d]pyrimidine (1.5 g, 0.00544 mol) and 4-fluoro-2-methylphenylamine (1.5 g, 0.0120 mol) were heated at 160° for 2.5 hours. Addition of methanol gave a solid which was collected by filtration and dried, (1.81 g). Recrystallization from ethanolic HCl/methanol gave the title compound as the hydrochloride salt, (1.44 g), m.p.216-218°.

$C_{20}H_{17}FN_4S$ HCl

Found C 59.96, H 4.54, N 13.85, S 8.19, $Cl^-$ 8.69%;
Requires C 59.92, H 4.53, N 13.98, S 8.00, $Cl^-$ 8.64%.

Example 7

2-(2-Methylphenylamino)-4-(phenylamino)thieno[2,3-d]pyrimidine hydrochloride

2-Chloro-4-(phenylamino)thieno[2,3-d]pyrimidine (3 g, 0.0115 mol) and o-toluidine (2.46 g, 0.023 mol) were heated at 140° for 3 hours. Addition of diethyl ether gave a solid which was collected by filtration and dried, (3.7 g). Recrystallization from ethanolic HCl gave the title compound as the hydrochloride salt, (1.67 g), m.p. 232-234°.

| | $C_{19}H_{16}N_4S$, HCl |
|---|---|
| Found | C 61.51, M 4.72, N 14.88, S 6.67, $Cl^-$9.51; |
| Requires | C 61.86, M 4.65, N 15.19, S 8.69, $Cl^-$9.61. |

Example 8

2,4-Bis(N-methylphenylamino)thieno(2,3-d)pyrimidine

2,4-Dichlorothieno[2,3-d]pyrimidine (2 g, 0.00975 mol) and N-methylaniline (5 ml) were heated in an oil bath at 150° for 2 hours. The reaction mixture was dissolved in chloroform (200 ml) and extracted with 2N hydrochloric acid (3x100 ml). The chloroform solution was then washed with sodium carbonate solution, dried over magnesium sulphate, filtered and evaporated to give an oil. The oil was purified by chromatography using dichloromethane as eluant. The solid obtained was recrystallized from ether/petroleum ether (b.p. 40-60°) to give the title compound, (1.2 g) m.p. 85-87°.

| | $C_{20}H_{18}N_4S$ |
|---|---|
| Found | C 68.96, H 5.36, N 16.06, S 9.14%; |
| Requires | C 69.34, H 5.24, N 16.17, S 9.26%. |

Example 9

2,4-Bis(2-methylphenylamino)thieno[2,3-d]pyrimidine

2,4-Dichlorothieno[2,3-d]pyrimidine (1.5 g, 0.0073 mol) and o-toluidine (5 ml) were heated in an oil bath at 160° for 2 hours. The reaction mixture was dissolved in chloroform (200 ml) and washed with 2N hydrochloric acid (3 x 100 ml), then sodium carbonate solution, dried over magnesium sulphate, filtered and evaporated under reduced pressure to give a solid, (1.98 g). The solid was purified by column chromatography using dichloromethane as eluant. The fractions containing the product were combined and evaporated under reduced pressure to give a solid. Recrystallization from diethyl ether/petroleum ether (b.p. 40-60°) gave the title compound, (1.0 g), m.p. 121-123°,

| | $C_{20}H_{18}N_4S$ |
|---|---|
| Found | C 69.48, H 5.36, N 16.20, S 9.44%; |
| Requires | C 69.34, H 5.24, N 16.17, S 9.26%. |

References for starting materials

2-Ethylmercapto-4-hydroxythieno[3,2-d]pyrimidine.
GB 1,309,182.
2,4-Dichlorothieno[2,3-d]pyrimidine.
GB 1,570,494.
2,4-Dichlorothieno[3,2-d]pyrimidine.
GB 1,309,182.

Biological Data.

(A) $H^+K^+$ATPase Activity.

The effects of a single high concentratration (100μM) of a compound of structure (I) on K-stimulated ATPase activity in lyophilised gastric vesicles was determined. Preferred compounds of structure (I) were also tested over a range of concentrations to determine $IC_{50}$ values.

(i) Preparation of lyophilised gastric vesicles (H/K-ATPase).

Lyophilised gastric vesicles were prepared from pig fundic mucosa after the method of Keeling et. al. (Biochem. Pharmacol., 34, 2967, 1985).

(ii) $K^+$-stimulated ATPase activity.

$K^+$-stimulated ATPase activity was determined at 37°C in the presence of the following : 10 mM Pipes/Tris buffer pH 7.0, 2 mM $MgSO_4$, 1 mM KCl, 2 mM $Na_2$ATP and 3-6 μg protein/ml lyophilised gastric vesicles. After incubation for 30 minutes, the inorganic phosphate hydrolysed from ATP was determined by the method of Yoda and Hokin (Biochem. Biophys. Res. Commun. 40, 880, 1970).

Compounds of structure (I) were dissolved in dimethylsulphoxide which up to the highest concentration used had no effect on $K^+$-stimulated ATPase activity.

The effect of the highest concentration of each compound of structure (I) on the recovery of a standard amount of inorganic phosphate was also determined.

(iii) Results.

The compounds of the example 1 to 6 and 8 had $IC_{50}$ values in the range of from 0.02 to 0.5 μM.

Example A

A tablet for oral administration is prepared by combining

| | mg /Tablet |
|---|---|
| Compound of structure (I) | 100 |
| lactose | 153 |
| Starch | 33 |
| crospovidone | 12 |
| microcrystalline cellulose | 30 |
| magnesium stearate | 2 |
| | 330 mg |

into a 9 mm tablet.

Example B

An injection for parenteral administration was prepared from the following

| | | %w:w |
|---|---|---|
| Compound of Structure (I) | | 0,50% (w:v) |
| 1M citric acid | | 30% (v:v) |
| sodium hydroxide (qs) | | to pH 3.2 |
| water for injection EP | to | 100 ml |

The compound of Structure (I) was dissolved in the citric acid and the pH slowly adjusted to pH 3.2 with the sodium hydroxide solution. The solution was then made up to 100 ml with water, sterilised by filtration and sealed into appropriately sized ampoules and vials.

## Claims

1. A compound of structure (I)

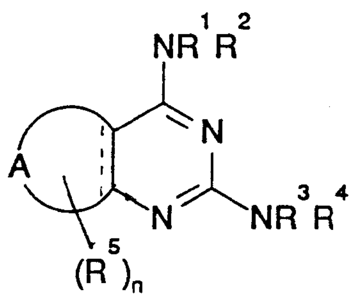

(I)

in which

$R^1$ and $R^2$ are the same, or different and are each hydrogen, $C_{1-4}$alkyl, $-(CH_2)_nAr$ in which n is 0 to 4 and Ar is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl;

$R^3$ and $R^4$ are the same or different and are each hydrogen, $C_{1-4}$alkyl, $(CH_2)_nAr^1$ in which n is 0 to 4 and $Ar^1$ is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl;

$R^5$ is hydroen, $C_{1-4}$alkyl, $C_{1-4}$alkoxy or $COC_{1-4}$alkyl;

n is 1 or 2;

A is -SCH=CH-, -CH=CHS- or =CHSCH=, and the dotted line indicates the presence of a double bond, when A is -SCH=CH- or -CH=CHS-;

or a pharmaceutically acceptable salt thereof; with the exception of 2-isobutylamino-4-isopropylaminothieno[2,3-d]pyrimidine.

2. A compound according to claim 1 in which one of $R^1$ and $R^2$ is $(CH_2)_nAr$ in which n is 0 to 4 and Ar is an optionally substituted phenyl group and the other is $C_{1-4}$alkyl.

3. A compound according to claim 2 in which n is O.

4. A compound according to claim 3 in which one of $R^3$ and $R^4$ is hydrogen and the other is $-(CH_2)_nAr^1$ in which n is 0 to 4 and $Ar^1$ is an optionally substituted phenyl group.

5. A compound according to claim 1 which is
2-(2-Methylphenylamino)-4-(N-methylphenylamino)thieno[3,2-d]pyrimidine.
2-(4-Methoxy-2-methylphenylamino)-4-(N-methylphenylamino)thieno[3,2-d]pyrimidine hydrochloride.
2-(4-fluoro-2-methylphenylamino)-4-(N-methylphenylamino)thieno[3,2-d]pyrimidine hydrochloride
2,4-Bis(N-methylphenylamino)thieno[3,2-d]pyrimidine
2-(2-Methylphenylamino)-4-(N-methylphenylamino)thieno[2,3-d]pyrimidine hydrochloride.
2-(4-Fluoro-2-methylphenylamino)-4-(N-methylphenylamino)thieno-[2,3-d]pyrimidine hydrochloride
2-(2-Methylphenylamino)-4-(phenylamino)thieno[2,3-d]pyrimidine hydrochloride
2,4-Bis(N-methylphenylamino)thieno(2,3-b)pyrimidine.
2,4-Bis(2-methylphenylamino)thieno(2,3-d)pyrimidine
or a pharmaceutically acceptable salt thereof,

6. A pharmaceutical composition comprising a compound according to any one of claims 1 to 5 and a pharmaceutical carrier.

7. A compound according to any one of claims 1 to 5 for use as a therapeutic agent.

8. A process for the preparation of a compound according to claim 1 which comprises :
(a) reaction of a compound of structure (II)

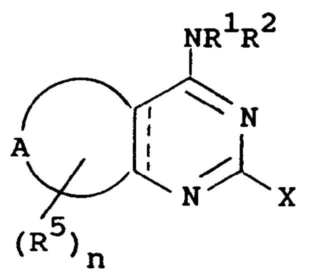

(II)

in which $R^1$, $R^2$, $R^5$, A and n are as described for structure (I) except that where necessary they are in protected form, and X is a group displaceable by an amine, with an amine of structure $R^3R^4NH$ in which $R^3$ and $R^4$ are as described for structure (I);
(b) reaction of a compound of structure (III)

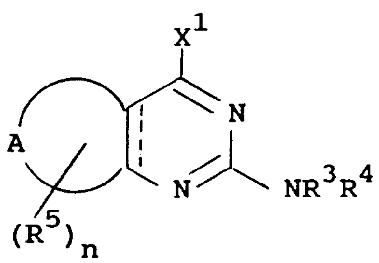

(III)

in which $R^3$, $R^4$, $R^5$, A and n are as described for structure (I) and $X^1$ is a group displaceable by an amine, with an amine of structure $R^1R^2NH$ in which $R^1$ and $R^2$ are as described for structure (I); or
(c) for compounds of structure (I) in which $NR^1R^2$ and $NR^3R^4$ are the same, reaction of a compound of structure (IV)

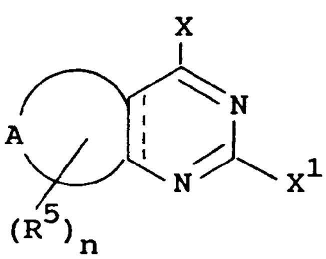

(IV)

in which $R^5$, A and n are as described for structure (I) and X and $X^1$ are groups displaceable by an amine, with an amine of structure $R^1R^2NH$ or $R^3R^4NH$.
and optionally thereafter,
° removing any protecting groups;
° forming a pharmaceutically acceptable salt.

9. Use of a compound of formula (IA):

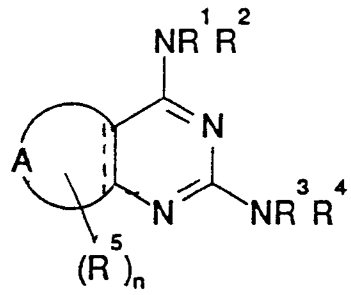

(IA)

in which

$R^1$ and $R^2$ are the same, or different and are each hydrogen, $C_{1-4}$alkyl, $-(CH_2)_nAr$ in which n is 0 to 4 and Ar is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^1$ and $R^2$ together with the nitrogen atom to which they are attached form a saturated or unsaturated ring optionally containing one or more further heteroatoms; and;

$R^3$ and $R^4$ are the same or different and are each hydrogen, $C_{1-4}$alkyl, $(CH_2)_nAr^1$ in which n is O to 4 and $Ar^1$ is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^3$ and $R^4$ together with the nitrogen atom to which they are attached form a saturated or unsaturated ring optionally containing one or more further heteroatoms.

$R^5$ is hydrogen, $C_{1-4}$alkyl, $C_{1-4}$alkoxy or $COC_{1-4}$alkyl;

n is 1 or 2;

A is -SCH=CH-, -CH=CHS- or =CHSCH=, and the dotted line indicates the presence of a double bond when A is -SCH=CH- or -CH=CHS-;

and pharmaceutically acceptable salts thereof in the manufacture of a medicament for the inhibition of gastric acid secretion.

**10.** Use of a compound of formula (IA) :

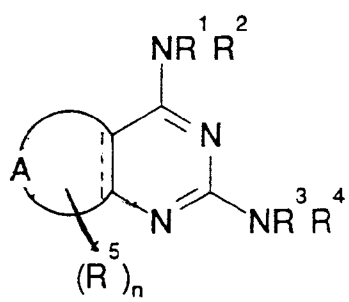

(IA)

in which

$R^1$ and $R^2$ are the same, or different and are each hydrogen, $C_{1-4}$alkyl, $-(CH_2)_nAr$ in which n is 0 to 4 and Ar is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^1$ and $R^2$ together with the nitrogen atom to which they are attached form a saturated or unsaturated ring optionally containing one or more further heteroatoms; and;

$R^3$ and $R^4$ are the same or different and are each hydrogen, $C_{1-4}$alkyl, $(CH_2)_nAr^1$ in which n is 0 to 4 and $Ar^1$ is a phenyl group which is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^3$ and $R^4$ together with the nitrogen atom to which they are attached form a saturated or unsaturated ring optionally containing one or more further heteroatoms.

$R^5$ is hydrogen, $C_{1-4}$alkyl, $C_{1-4}$alkoxy or $COC_{1-4}$alkyl;
n is 1 or 2;
A is -SCH=CH-, -CH=CHS- or =CHSCH=, and the dotted line indicates the presence of a double bond when A is -SCH=CH- or -CH=CHS-;
and pharmaceutically acceptable salts thereof in the manufacture of a medicament for the inhibition of bone resorption.

## Patentansprüche

1. Verbindung der Struktur (I)

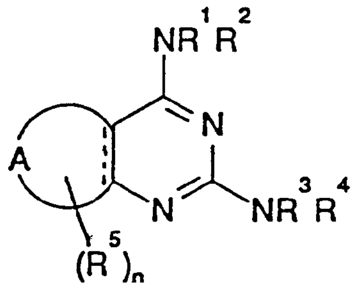

(I)

in der
$R^1$ und $R^2$ gleich oder verschieden sind und jeweils Wasserstoffatome, $C_{1-4}$-Alkyl- oder $-(CH_2)_n$Ar-Reste bedeuten, wobei n einen Wert von 0 bis 4 hat und Ar eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen;
$R^3$ und $R^4$ gleich oder verschieden sind und jeweils Wasserstoffatome, $C_{1-4}$-Alkyl- oder $(CH_2)_n$$Ar^1$-Reste bedeuten, wobei n einen Wert von 0 bis 4 hat und $Ar^1$ eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen;
$R^5$ ein Wasserstoffatom, ein $C_{1-4}$-Alkyl-, $C_{1-4}$-Alkoxy- oder $COC_{1-4}$-Alkylrest ist;
n den Wert 1 oder 2 hat;
A eine Gruppe -SCH=CH-, -CH=CHS- oder =CHSCH= ist und die gepunktete Linie die Gegenwart einer Doppelbindung bedeutet, wenn A eine Gruppe -SCH=CH- oder -CH=CHS- ist;
oder ein pharmazeutisch verträgliches Salz davon, mit Ausnahme von 2-Isobutylamino-4-isopropylaminothieno[2,3-d] pyrimidin.

2. Verbindung nach Anspruch 1, in der einer der Reste $R^1$ und $R^2$ ein $(CH_2)_n$Ar-Rest ist, wobei n einen Wert von 0 bis 4 hat und Ar eine gegebenenfalls substituierte Phenylgruppe ist und der andere Rest ein $C_{1-4}$-Alkylrest ist.

3. Verbindung nach Anspruch 2, wobei n den Wert 0 hat.

4. Verbindung nach Anspruch 3, in der einer der Reste $R^3$ und $R^4$ ein Wasserstoffatom ist und der andere ein $-(CH_2)_n$$Ar^1$-Rest ist, wobei n einen Wert von 0 bis 4 hat und $Ar^1$ eine gegebenenfalls substituierte Phenylgruppe ist.

5. Verbindung nach Anspruch 1, nämlich
2-(2-Methylphenylamino)-4-(N-methylphenylamino)thieno[3,2-d]pyrimidin,
2-(4-Methoxy-2-methylphenylamino)-4-(N-methylphenylamino)-thieno[3,2-d]pyrimidin-hydrochlorid,
2-(4-Fluor-2-methylphenylamino)-4-(N-methylphenylamino)thieno[3,2-d]pyrimidin-hydrochlorid,
2,4-Bis(N-methylphenylamino)thieno[3,2-d]pyrimidin,
2-(2-Methylphenylamino)-4-(N-methylphenylamino)thieno-[2,3-d]pyrimidin-hydrochlorid,
2-(4-Fluor-2-methylphenylamino)-4-(N-methylphenylamino)thieno-[2,3-d]pyrimidin-hydrochlorid,

2-(2-Methylphenylamino)-4-(phenylamino)thieno[2,3-d]-pyrimidin-hydrochlorid, 2,4-Bis(N-methylphenylamino)thieno(2,3-b)pyrimidin oder 2,4-Bis(2-methylphenylamino)thieno(2,3-d)pyrimidin oder ein pharmazeutisch verträgliches Salz davon.

6. Arzneimittel, umfassend eine Verbindung nach einem der Ansprüche 1 bis 5 und einen pharmazeutischen Träger.

7. Verbindung nach einem der Ansprüche 1 bis 5 zur Verwendung als Therapeutikum.

8. Verfahren zur Herstellung einer Verbindung nach Anspruch 1, umfassend:
(a) Umsetzen einer Verbindung der Struktur (II)

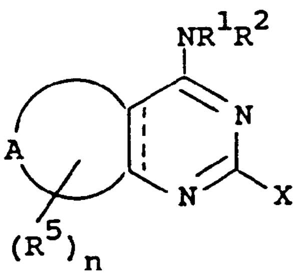

(II)

in der $R^1$, $R^2$, $R^5$, A und n die gleiche Bedeutung wie bei Struktur (I) haben, mit der Ausnahme daß sie, wenn nötig in der geschützten Form vorliegen können und X eine durch ein Amin ersetzbare Gruppe ist, mit einem Amin der Struktur $R^3R^4NH$, in der $R^3$ und $R^4$ die gleiche Bedeutung wie bei Struktur (I) haben;
(b) Umsetzen einer Verbindung der Struktur (III)

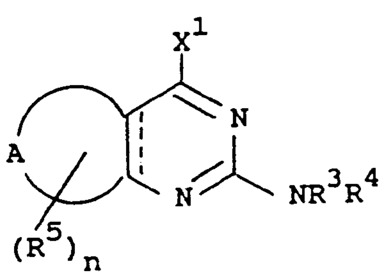

(III)

in der $R^3$, $R^4$, $R^5$, A und n die gleiche Bedeutung wie bei Struktur (I) haben und $X^1$ eine durch ein Amin ersetzbare Gruppe ist, mit einem Amin der Struktur $R^1R^2NH$, in der $R^1$ und $R^2$ die gleiche Bedeutung wie bei Struktur (1) haben; oder
(c) für Verbindungen der Struktur (I), in denen $NR^1R^2$ und $NR^3R^4$ gleich sind, Umsetzen einer Verbindung der Struktur (IV)

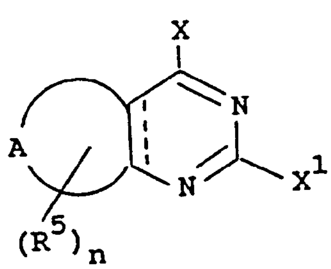

(IV)

in der $R^5$, A und n die gleiche Bedeutung wie bei Struktur (I) haben und X und $X^1$ durch ein Amin ersetzbare Gruppen sind, mit einem Amin der Struktur $R^1R^2NH$ oder $R^3R^4NH$, und gegebenenfalls anschließend

Entfernen der Schutzgruppen;
Erzeugen eines pharmazeutisch verträglichen Salzes

9. Verwendung einer Verbindung der Formel (IA)

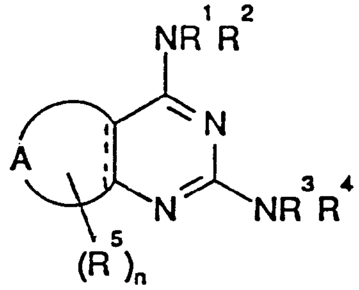

(IA)

in der
$R^1$ und $R^2$ gleich oder verschieden sind und jeweils Wasserstoffatome, $C_{1-4}$-Alkyl- oder $-(CH_2)_nAr$-Reste bedeuten, wobei n einen Wert von 0 bis 4 hat, und Ar eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen, oder $R^1$ und $R^2$ zusammen mit dem Stickstoffatom, an das sie gebunden sind, einen gesättigten oder ungesättigten Ring bilden, der gegebenenfalls ein oder mehrere weitere Heteroatome enthält, und
$R^3$ und $R^4$ gleich oder verschieden sind und jeweils Wasserstoffatome, $C_{1-4}$-Alkyl- oder $(CH_2)_nAr^1$-Reste bedeuten, wobei n einen Wert von 0 bis 4 hat und $Ar^1$ eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen, oder $R^3$ und $R^4$ zusammen mit dem Stickstoffatom, an das sie gebunden sind, einen gesättigten oder ungesättigten Ring bilden, der gegebenenfalls ein oder mehrere weitere Heteroatome enthält,
$R^5$ ein Wasserstoffatom, ein $C_{1-4}$-Alkyl-, $C_{1-4}$-Alkoxy oder $COC_{1-4}$-Alkylrest ist;
n den Wert 1 oder 2 hat;
A eine Gruppe -SCH=CH-, -CH=CHS- oder =CHSCH= ist und die gepunktete Linie die Gegenwart einer Doppelbindung anzeigt, wenn A eine Gruppe -SCH=CH- oder -CH=CHS- ist;
und pharmazeutisch verträgliche Salze davon für die Herstellung eines Arzneimittels zur Hemmung der Magensäuresekretion.

10. Verwendung einer Verbindung der Formel (IA)

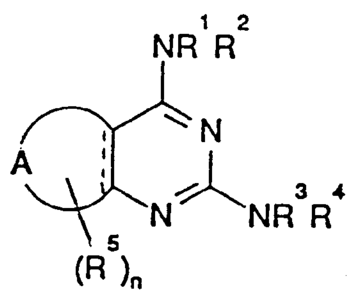

(IA)

in der
$R^1$ und $R^2$ gleich oder verschieden sind und jeweils Wasserstoffatome, $C_{1-4}$-Alkyl- oder $-(CH_2)_nAr$-Reste bedeuten, wobei n einen Wert von 0 bis 4 hat, und Ar eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen, oder $R^1$ und $R^2$ zusammen mit dem Stickstoffatom, an das sie gebunden sind, einen gesättigten oder ungesättigten Ring bilden, der gegebenenfalls ein oder mehrere weitere Heteroatome enthält, und

$R^3$ und $R^4$ gleich oder verschieden sind und jeweils Wasserstoffatome, $C_{1-4}$-Alkyl- oder $(CH_2)_nAr^1$-Reste bedeuten, wobei n einen Wert von 0 bis 4 hat und $Ar^1$ eine unsubstituierte oder mit 1 bis 3 Substituenten substituierte Phenylgruppe ist, welche ausgewählt sind aus $C_{1-4}$-Alkyl-, $C_{1-4}$-Alkoxy-, $C_{1-4}$-Alkylthioresten, Halogenatomen, Cyano-, Amino-, Hydroxyl-, Carbamoyl-, Carboxylgruppen, $C_{1-4}$-Alkanoylresten oder Trifluormethylgruppen, oder $R^3$ und $R^4$ zusammen mit dem Stickstoffatom, an das sie gebunden sind, einen gesättigten oder ungesättigten Ring bilden, der gegebenenfalls ein oder mehrere weitere Heteroatome enthält,
$R^5$ ein Wasserstoffatom, ein $C_{1-4}$-Alkyl-, $C_{1-4}$-Alkoxy- oder $COC_{1-4}$-Alkylrest ist;
n den Wert 1 oder 2 hat;
A eine Gruppe -SCH=CH-, -CH=CHS- oder =CHSCH= ist und die gepunktete Linie die Gegenwart einer Doppelbindung anzeigt, wenn A eine Gruppe -SCH=CH- oder -CH=CHS- ist;
und pharmazeutisch verträgliche Salze davon für die Herstellung eines Arzneimittels zur Hemmung der Knochenresorption.

## Revendications

1. Composé de structure (I)

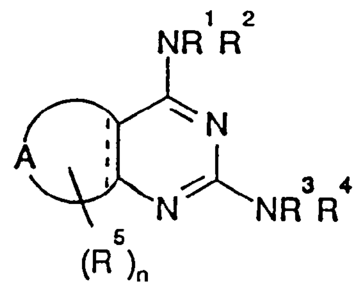

(I)

dans laquelle

$R^1$ et $R^2$ sont identiques ou différents et représentent chacun un atome d'hydrogène ou un groupe alkyle en $C_{1-4}$ ou $-(CH_2)_nAr$ où n vaut de 0 à 4 et Ar représente un groupe phényle non substitué ou substitué par 1 à 3 substituants choisis parmi les groupes alkyle en $C_{1-4}$ , alcoxy en $C_{1-4}$, alkylthio en $C_{1-4}$, halogéno, cyano, amino, hydroxy, carbamoyle, carboxy, alcanoyle en $C_{1-4}$ ou trifluorométhyle ;

$R^3$ et $R^4$ sont identiques ou différents et représentent chacun un atome d'hydrogène ou un groupe alkyle en $C_{1-4}$ ou $-(CH_2)_nAr^1$ où n vaut de 0 à 4 et $Ar^1$ représente un groupe phényle non substitué ou substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle ;

$R^5$ représente un atome d'hydrogène ou un groupe alkyle en $C_{1-4}$, alcoxy en $C_{1-4}$ ou CO-alkyle en $C_{1-4}$ ;

n vaut 1 ou 2 ;

A représente -SCH=CH-, -CH=CHS- ou =CHSCH-, et la ligne en pointillé indique la présence d'une double liaison quand A représente -SCH=CH- ou -CH=CHS- ;

ou un sel pharmaceutiquement acceptable de celui-ci, à l'exception de la 2-isobutylamino-4-isopropylaminothiéno[2,3-d]pyrimidine.

2. Composé selon la revendication 1, dans lequel un des $R^1$ et $R^2$ est $(CH_2)_nAr$, où n vaut de 0 à 4 et Ar représente un groupe phényle éventuellement substitué, et l'autre $R^1$ ou $R^2$ est un groupe alkyle en $C_{1-4}$.

3. Composé selon la revendication 2, dans lequel n vaut 0.

4. Composé selon la revendication 3, dans lequel un des $R^3$ et $R^4$ est un atome d'hydrogène, et l'autre $R^3$ ou $R^4$ est $-(CH_2)_nAr^1$, où n vaut de 0 à 4 et $Ar^1$ représente un groupe phényle éventuellement substitué.

5. Composé selon la revendication 1, qui est :
la 2-(2-méthylphénylamino)-4-(N-méthylphénylamino)thiéno[3,2-d]pyrimidine ;
le chlorhydrate de 2-(4-méthoxy-2-méthylphénylamino)-4-(N-méthylphénylamino)thiéno[3,2-d]pyrimidine ;
le chlorhydrate de 2-(4-fluoro-2-méthylphénylamino)-4-(N-méthylphénylamino)thiéno[3,2-d]pyrimidine ;
la 2,4-bis(N-méthylphénylamino)thiéno[3,2-d]pyrimidine ;
le chlorhydrate de 2-(2-méthylphénylamino)-4-(N-méthylphénylamino)thiéno[2,3-d]pyrimidine ;
le chlorhydrate de 2-(4-fluoro-2-méthylphénylamino)-4-(N-méthylphénylamino)thiéno[2,3-d]pyrimidine ;
le chlorhydrate de 2-(2-méthylphénylamino)-4-(phénylamino)thiéno[2,3-d]pyrimidine ;
la 2,4-bis(N-méthylphénylamino)thiéno(2,3-b)pyrimidine ;
la 2,4-bis(2-méthylphénylamino)thiéno(2,3-d)pyrimidine ;
ou un sel pharmaceutiquement acceptable de ces composés.

6. Composition pharmaceutique comprenant un composé selon une quelconque des revendications 1 à 5 et un excipient pharmaceutique.

7. Composé selon une quelconque des revendications 1 à 5 pour une utilisation comme agent thérapeutique.

8. Procédé de préparation d'un composé selon la revendication 1, comprenant :
(a) la réaction d'un composé de structure (II)

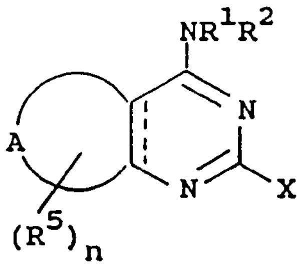

(II)

dans laquelle $R^1$, $R^2$, $R^5$, A et n sont tels que décrits pour la structure (I), sauf qu'ils sont sous une forme protégée si cela est nécessaire, et X est un groupe pouvant être remplacé par une amine,
avec une amine de structure $R^3R^4NH$, dans laquelle $R^3$ et $R^4$ sont tels que décrits pour la structure (I) ;
(b) la réaction d'un composé de structure (III)

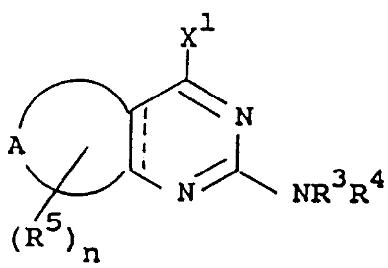

(III)

dans laquelle $R^3$, $R^4$, $R^5$, A et n sont tels que décrits pour la structure (I), et $X^1$ est un groupe pouvant être remplacé par une amine,
avec une amine de structure $R^1R^2NH$, dans laquelle $R^1$ et $R^2$ sont tels que décrits pour la structure (I) ;
ou
(c) pour des composés de structure (I) où $NR^1R^2$ et $NR^3R^4$ sont identiques, la réaction d'un composé de structure (IV)

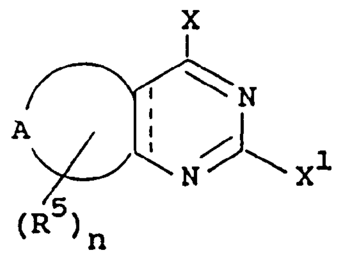
(IV)

dans laquelle $R^5$, A et n sont tels que décrits pour la structure (I), et X et $X^1$ sont des groupes pouvant être remplacés par une amine,

avec une amine de structure $R^1R^2NH$ ou $R^3R^4NH$,

et ensuite, éventuellement :

- éliminer tous les groupes protecteurs ;
- former un sel pharmaceutiquement acceptable.

**9.** Utilisation d'un composé de formule (IA) :

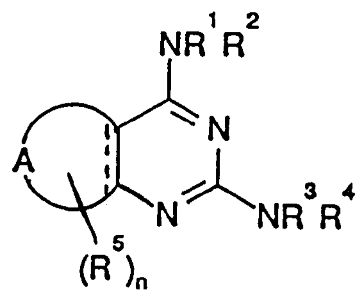
(IA)

dans laquelle

$R^1$ et $R^2$ sont identiques ou différents et représentent chacun un atome d'hydrogène ou un groupe alkyle en $C_{1-4}$ ou $-(CH_2)_nAr$, où n vaut de 0 à 4 et Ar représente un groupe phényle non substitué ou substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou bien $R^1$ et $R^2$, conjointement avec l'atome d'azote auquel ils sont liés, forment un composé cyclique saturé ou non saturé contenant éventuellement un ou plusieurs autres héréroatomes ; et

$R^3$ et $R^4$ sont identiques ou différents et représentent chacun un atome d'hydrogène ou un groupe alkyle en $C_{1-4}$ ou $-(CH_2)_nAr^1$ où n vaut de 0 à 4 et $Ar^1$ représente un groupe phényle non substitué ou substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou bien $R^3$ et $R^4$, conjointement avec l'atome d'azote auquel ils sont liés, forment un composé cyclique saturé ou non saturé contenant éventuellement un ou plusieurs autres héréroatomes ;

$R^5$ représente un atome d'hydrogène ou un groupe alkyle en $C_{1-4}$, alcoxy en $C_{1-4}$ ou CO-alkyle en $C_{1-4}$ ;

n vaut 1 ou 2 ;

A représente -SCH=CH-, -CH=CHS- ou =CHSCH-, et la ligne en pointillé indique la présence d'une double liaison quand A représente -SCH=CH- ou -CH=CHS- ;

et ses sels pharmaceutiquement acceptables, pour la fabrication d'un médicament pour inhiber la sécrétion d'acide gastrique.

**10.** Utilisation d'un composé de formule (IA) :

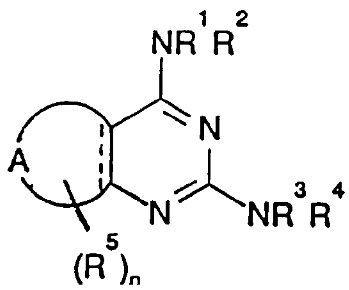

(IA)

dans laquelle

$R^1$ et $R^2$ sont identiques ou différents et représentent chacun un atome d'hydrogène ou un groupe alkyle en $C_{1-4}$ ou $-(CH_2)_nAr$, où n vaut de 0 à 4 et Ar représente un groupe phényle non substitué ou substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou bien $R^1$ et $R^2$, conjointement avec l'atome d'azote auquel ils sont liés, forment un composé cyclique saturé ou non saturé contenant éventuellement un ou plusieurs autres héréroatomes ; et

$R^3$ et $R^4$ sont identiques ou différents et représentent chacun un atome d'hydrogène ou un groupe alkyle en $C_{1-4}$ ou $-(CH_2)_nAr^1$ où n vaut de 0 à 4 et $Ar^1$ représente un groupe phényle non substitué ou substitué par 1 à 3 substituants choisis parmi les groupes alkyle en $C_{1-4}$, alcoxy en $C_{1-4}$, alkylthio en $C_{1-4}$, halogéno, cyano, amino, hydroxy, carbamoyle, carboxy, alcanoyle en $C_{1-4}$ ou trifluorométhyle, ou bien $R^3$ et $R^4$, conjointement avec l'atome d'azote auquel ils sont liés, forment un composé cyclique saturé ou non saturé contenant éventuellement un ou plusieurs autres héréroatomes ;

$R^5$ représente un atome d'hydrogène ou un groupe alkyle en $C_{1-4}$, alcoxy en $C_{1-4}$ ou CO-alkyle en $C_{1-4}$ ;

n vaut 1 ou 2 ;

A représente -SCH=CH-, -CH=CHS- ou =CHSCH-, et la ligne en pointillé indique la présence d'une double liaison quand A représente -SCH=CH- ou -CH=CHS- ;

et ses sels pharmaceutiquement acceptables, pour la fabrication d'un médicament pour inhiber la résorption osseuse.